# EUROPEAN PATENT SPECIFICATION

(11) **EP 2 938 320 B1**
(45) Date of publication and mention of the grant of the patent: **12.09.2018**
(21) Application number: 13801584.7
(22) Date of filing: 06.12.2013
(51) Int. Cl.: A61K 8/36, A61K 8/73, A61Q 17/04, A61K 8/81, A61K 8/90

(54) **A SUNSCREEN COMPOSITION COMPRISING FATTY ACID AND NON-IONIC LINEAR POLYMER**
SONNENSCHUTZZUSAMMENSETZUNG ENTHALTEND EINE FETTSAÜRE UND EIN NICHT-IONISCHES, LINEARES POLYMER
COMPOSITION D'ÉCRAN SOLAIRE COMPRENANT UN ACIDE GRAS ET UN POLYMÈRE LINÉAIRE NON-IONIQUE

(30) Priority: 27.12.2012 EP 12199401
(43) Date of publication of application: 04.11.2015
(73) Proprietor: UNILEVER N.V., 3013 AL Rotterdam (NL); Unilever PLC, London, Greater London EC4Y 0DY (GB)
(72) Inventor: KUNJUPILLAI, Balu, Bangalore 560 066 (IN); PERUMAL, Rajkumar, Bangalore 560 066 (IN); RASTOGI, Abhishek, Bangalore 560 066 (IN)
(74) Representative: Corsten, Michael Allan
(86) International application number: PCT/EP2013/075836
(87) International publication number: WO 2014/102050

(56) References cited:
- WO-A2-2011/147738
- DE-A1- 10 214 059
- JP-A- 2009 263 275
- US-A- 4 699 779
- US-A- 5 427 774
- US-A1- 2004 180 011
- US-A1- 2011 020 253
- US-A1- 2011 142 770
- US-A1- 2012 251 481

## Description

### TECHNICAL FIELD

The invention relates to a sunscreen composition, especially to a composition that gives good Sun Protection Factor (SPF).

### BACKGROUND OF THE INVENTION

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore, it is desirable to protect the skin and other keratinous substrates of the human body from the harmful effects of both, UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. Many UV-B sunscreens are also known and approved for safe use in personal care compositions for protection from UV-B radiation. Many cosmetic manufacturers prefer to include both UV-A and UV-B sunscreens in photoprotective compositions so as to provide protection over the entire range of UV radiation. Organic sunscreens act by absorbing the UV radiation from the sun and undergo chemical transformation leading to emitting radiation at a different wavelength. Due to the chemical nature of the mode of action, many organic sunscreen are unstable and protection over skin is often achieved only for a few minutes or hours.

Another approach is to include inorganic particles like zinc oxide or titanium di oxide which act as sun blocks that prevent the sun's rays from reaching the skin and/ or scatter the incident solar radiation. Inorganic particles, due to their mode of action act across the radiation spectrum but have the inherent disadvantage of giving the skin an artificially whitish look.

Sun protection Factor (SPF) is a commonly measured attribute of photoprotective compositions which indicate the protection that the skin gets from exposure to both UV-B and UV-A radiation. Cosmetic manufacturers try to provide consumers with products having higher and higher SPF. One of the ways of achieving this is to incorporate high levels of UV-A and UV-B sunscreens. One disadvantage of this approach is the high cost associated with incorporation of high levels of sunscreens which are expensive. Further, there may be safety and regulatory limitations on the upper limit of incorporation of these sunscreens. Sensory properties are also reported to get affected on incorporation of high levels of sunscreens. Hence, there is a problem of achieving high SPF/ UVAPF while keeping the total amount of sunscreens in the compositions relatively low.

To achieve high SPF from sunscreen compositions, it has been necessary in the past to include high amounts of organic sunscreens. Organic sunscreens at higher than 8% and often higher than 12% are generally included in order to get an in-vitro SPF of higher than 15. The present inventors have been working on alternative approaches to provide high SPF at low sunscreen concentrations and with their knowledge of polymers and structures of cosmetic bases approached the problems of high SPF from this angle. They have found that specific classes of polymers when included in a cosmetically acceptable base comprising selective amount of fatty acid, provide enhanced sun protection.
WO07/140442 (Nutrogena) relates to clear sunscreen compositions that include an organic sunscreen and an alcohol. In certain embodiments, the compositions include a rheology modifier such as a non-ionic polymer and an anti-pilling agent such as a cationic polymer.
WO11/163589 (Hercules) relates to a a personal care composition additive for use on keratin substrates in order to provide long lasting benefits to the keratin substrate such as in conditioning systems, such as 2/1 shampoo's, leave-on and rinse off conditioners, for hair and skin, or for imparting greater water resistance to such personal care compositions as sunscreens or cosmetics. US 2011/020253A1 discloses in Claim 25 a sunscreen composition comprising UVA sunscreen (benzophenone-3), UVB sunscreen (3,3,5-Trimethylcyclohexyl 2-hydroxybenzoate, Octyl methoxycinnamate and Octyl salicylate), fatty acid (dodecanoic acid) and water. The compositions disclosed in the prior art do not disclose how it is possible to provide high SPF by way of inclusion of specific non-ionic polymers in a fatty acid containing personal care composition.
It is therefore an object of the present invention to obviate at least some drawbacks of the prior art and provide high SPF photo-protective personal care composition.

### SUMMARY OF THE INVENTION

According to the first aspect of the invention there is provided a sunscreen composition comprising at the most 12% organic sunscreens comprising
(i) 0.1 to 5% of a UVA sunscreen;
(ii) 0.1 to 10% of a UVB sunscreen;
(iii) 0.1 to 10% of a non-ionic linear polymer having a molecular weight higher than 8000 and HLB value from 7 to 18 selected from the group of poly vinyl alcohol, modified cellulose and poly(propylene oxide-co-ethylene oxide);
(iv) 4 to 25 % fatty acid; and
(v) 40 to 80% water, characterized in that:
   - the UVA sunscreen comprises a dibenzoylmethane, triazine, or triazone derivative; and
   - the non-ionic linear polymer (iii) comprises poly vinyl alcohol.

### DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.
"Sunscreen composition" as used herein, is meant to include a composition for topical application to skin and/or hair of mammals, especially humans particularly for sunscreen benefits. Such a composition may be generally classified as leave-on or rinse off, and includes any product applied to a human body primarily for sun protection but may be used also for improving appearance, cleansing, odor control or general aesthetics. The composition of the present invention can be in the form of a liquid, lotion, cream, foam, scrub, gel, soap bar or toner, or applied with an implement or via a face mask, pad or patch. Non-limiting examples of personal care compositions include leave-on skin lotions and creams, shampoos, conditioners, shower gels, toilet bars, antiperspirants, deodorants, dental products, shave creams, depilatories, lipsticks, foundations, mascara, sunless tanners and sunscreen lotions. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.
The composition of the invention comprises a UV-A sunscreen, a UV-B sunscreen, a non-ionic polymer satisfying specific criteria in an oil-in-water base comprising fatty acid. By enhanced photoprotection is meant that the in-vitro sun protection factor (SPF as measured using the ISO/WD 24445 standard protocol) is higher than 15.

The composition of the invention comprises a UV-A sunscreen comprising a dibenzoylmethane, triazine, or triazone derivative. A benzophenone derivative may optionally be included. More preferred UV-A sunscreen is a dibenzoylmethane or its derivative. Preferred dibenzoylmethane derivative is selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. UV-A sunscreen is present in 0.1 to 5%, preferably 0.2 to 5%, more preferably 0.4 to 3%, by weight of the composition.

The composition of the invention comprises 0.1 to 10%, preferably from 0.5 to 6%, more preferably 1 to 5%, a UV-B organic sunscreen by weight of the composition. UV-B sunscreen is preferably selected from the class of benzophenones, anthranilates, salicylates, cinnamates, camphores, benzylidene malonate; triazone or derivatives thereof.

The UV-B organic sunscreen is preferably oil-soluble. UV-B sunscreen is preferably selected from the class of cinnamic acid, salicylic acid, diphenyl acrylic acid or derivatives thereof. A few of the preferred oil soluble UV-B sunscreens which are commercially available and useful for inclusion in the composition of the invention are Octisalate™ (octyl salicylate), Homosalate™ (3,3,5-trimethyleyclohexyl 2-hydroxybenzoate), Neo Heliopan™ (a range of organic UV filters including ethylhexyl methoxycinnamate (Neo Heliopan AV) and ethylhexyl salicylate (Neo Heliopan OS)), Octocrylene™ (2-ethylhexyl 2-cyano-3,3-diphenyl-2-propenoate) or Parsol MCX™ (2-ethylhexyl-4-methoxycinnamate). According to a particularly preferred aspect of the invention the UVB sunscreen is 2-ethylhexyl-4-methoxycinnamate. According to another particularly preferred aspect of the invention the oil soluble UVB sunscreen is 2-ethylhexyl 2-cyano-3,3-diphenyl-2-propenoate.

An especially preferred oil soluble UVB sunscreen is 2-ethylhexyl 2-cyano-3,3-diphenyl-2-propenoate which is commercially available as Octocrylene™. Octocrylene has the chemical formula 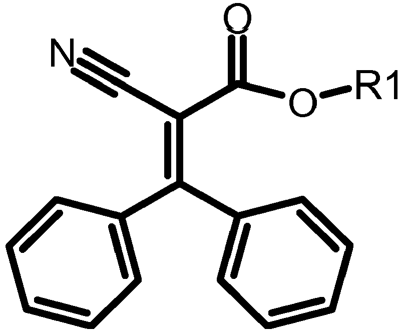 and R1 is a straight or branched chain C1-C30 alkyl group.
The total amount of organic sunscreen in the composition of the invention is preferably at the most 8%.
The sunscreen composition comprises a non-ionic polymer having a molecular weight higher than 8000 and HLB value from 7 to 18 selected from poly vinyl alcohol, and further optionally modified cellulose and poly(propylene oxide-co-ethylene oxide). The non-ionic polymer preferably has a molecular weight of at most 400000, more preferably at most 100000. The HLB value of the non-ionic polymer is from 7 to 18, preferably in the range of 15 to 18.
HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
- A value <10 : Lipid soluble (water insoluble)
- A value >10 : Water soluble
- A value from 4 to 8 indicates an anti-foaming agent
- A value from 7 to 11 indicates a W/O (water in oil) emulsifier
- A value from 12 to 16 indicates oil in water emulsion
- A value from 11 to 14 indicates a wetting agent
- A value from 12 to 15 is typical of detergents
- A value of 16 to 20 indicates a solubiliser or hydrotrope.

The non-ionic polymer is present in 0.1 to 10%, preferably 0.5 to 4% by weight of the composition. The surface tension of a 1% solution of the non-ionic polymer in water at 25°C is preferably higher than 40 dynes/cm i.e. non-ionic surfactants are not included in the class of non-ionic polymers claimed in the composition of the invention.

When the non-ionic polymer is a modified cellulose it is preferably a methyl cellulose or hydroxyethyl cellulose.

The composition of the invention comprises 4 to 25%, preferably 8 to 20%, fatty acid by weight of the composition. In a preferred aspect the composition may include 0.1 to 10% soap. The cosmetically acceptable bases are preferably in a cream, lotion, or emulsion format. A more preferred format is a cream or lotion, further more preferred format is a vanishing cream. The fatty acids may be saturated or unsaturated fatty acids. The base preferably comprises 0.1 to 3% soap. C₁₂ to C₂₀ fatty acids are especially preferred in vanishing cream bases, further more preferred being C₁₄ to C₁₈ fatty acids. In creams, the fatty acid is preferably substantially a mixture of stearic acid and palmitic acid. Soaps in the vanishing cream base include alkali metal salt of fatty acids, like sodium or potassium salts. The soap is preferably the potassium salt of the fatty acid mixture. The fatty acid in vanishing cream base is often prepared using hystric acid which is substantially (generally about 90 to 95%) a mixture of stearic acid and palmitic acid. Thus, inclusion of hystric acid and its soap to prepare the vanishing cream base is within the scope of the present invention. It is particularly preferred that the composition comprises at least 6%, preferably at least 10%, more preferably at least 12% fatty acid. The cosmetically acceptable base is usually from 10 to 99.9%, preferably from 50 to 99% by weight of the composition. The cosmetically acceptable base includes water. Water is included in 40 to 80% more preferably 50 to 80% by weight of the composition.
The sunscreen composition of the invention is for photo protecting the human epidermis or hair against the damaging effect of solar irradiation, as antisun/sunscreen composition or as makeup product. Such compositions can, in particular, be provided in the form of a lotion, a thickened lotion, a gel, a cream, cleansing milk, an ointment, a powder or a solid tube stick and may optionally be packaged as an aerosol and may be provided in the form of a mousse, foam or a spray.
The personal care compositions of the invention can also contain usual cosmetic adjuvants and skin care additives commonly employed in skin care products such as liquid or solid emollients, silicone oils, emulsifiers, solvents, humectants, polymeric or inorganic thickeners, powders, pigments (example clay mineral, barium sulfate, or pearl pigments, for example silver or gold, or any iris foil pearl pigment, having an interference color of red, orange, green, blue, or, purple (including any iris foil pearl pigments covered with inorganic pigments, organic pigments, laked pigments, etc.), bismuth oxychloride, bismuth oxychloride coated mica,) organic or inorganic sunscreens with and without photostabiliser, skin lightening agents, skin conditioners, optical brighteners, propellants, healing agents (example allantoin), cooling agents (example urea, menthol, menthyl lactate, frescolate), antiseptic agents and other specific skin-benefit actives, skin care actives such as skin lightening actives, antiaging, antiacne, antibacterials, antiperspirant agents etc,. The vehicle may also further include adjuncts such as antioxidants, perfumes, opacifiers, preservatives, colorants and buffers. The necessary amounts of the cosmetic and dermatological adjuvants and additives, based on the desired product, can be chosen by the skilled person.

Vitamins, which act as skin-lightening ingredients can be advantageously included in the composition to provide for additional skin lightening effects. These include vitamin B3, vitamin B6, vitamin C, vitamin A or their precursors and cosmetically acceptable derivatives. Mixtures of the vitamins can also be employed in the composition of the invention. When present, these vitamins are used in the range of 0.01 to 10.0% by weight of said composition.

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palpitate, silicone oils such as dimethylpolysiloxane, organomodified silicones such as cetyl dimethicone, steryl dimethicones; cross-linked silicone elastomers/resins; organo-modified cross-linked silicone elastomers/resins di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate; Propellants, such as propane, butane, isobutane, dimethyl ether, carbon dioxide, nitrous oxide; Solvents, such as ethyl alcohol, isopropanol, acetone/ ethylene glycol monoethyl ether, diethylene glycol monobutyl ether, diethylene glycol monoethyl ether; Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate; Plant extracts such as those from genus Rubia, Symplocus, Curcuma and various perfume/fragrance ingredients may also be included in the composition at ranges from 0.001 to 40.0% by weight of the composition. The emollient is preferably present in an amount from about 1 to about 20%, preferably from about 2 to about 15%, and most preferably from about 4 to about 10% of the total weight of the composition.

The preservatives and antioxidants are preferably present in an amount ranging from about 0.01 to about 10% of the total weight of the composition. Preferably, the preservatives and/or antioxidants are present in an amount varying from about 0.1 to about 1%

Additional emulsifiers may be used to enhance the stability of the oil in water emulsion composition of the invention but these are not required as the silicone polymer has both the hydrophilic group and the hydrophobic group to not only provide the spreading desired but also the emulsifying property to enable forming stable oil in water emulsion. However small amount of additional emulsifiers may be present. When present, these are selected from the group of non-ionic surfactants e.g. fatty alcohol ethoxylates (e.g. Brij 35), alkyl phenol ethoxylates (Triton group) and polyoxyethylene sorbitan alkyl esters (e.g. Tween 20). They are preferably present in less than 5%, more preferably less than 3%, and most preferably less than 2% by weight of the composition. Optimally they are absent from the composition.

The oily phase of the compositions according to the present invention may also contain natural vegetable or animal waxes such as bee wax, china wax, bumblebee wax and other waxes of insects as well as shea butter.

The aqueous phase of the formulations of the present invention may contain the usual cosmetic additives such as alcohols, especially lower alcohols, preferably ethanol and so or isopropanol, low alkyl diols or polyols and their ethers, preferably propyleneglycol, glycerine, ethyleneglycol, ethylene glycol monoethyl or monobutyl ether, electrolytes and especially, one or more thickeners. Thickeners that may be used in formulations of the present invention include the family of silicon dioxide, magnesium and/or aluminum silicates, polysaccharides and their derivatives such as hyaluronic acid, xanthan gum, hydroxypropyl cellulose, acrylate copolymers, preferably a polyacrylate of the family of carbopols, such as carbopols of type 980, 981, 1382, 2984, 59S4.

Moisturizing agents, such as humectants, may be incorporated into the compositions according to the present invention to reduce the trans- epidermal water loss (TEWL) of the horny layer of the skin. Suitable humectants include glycerin, lactic acid, pyrrolidone carbonic acid, urea, polyethylene glycol, polypropylene glycol, sorbitol, PEG- 400, and mixtures thereof. Additional suitable moisturizers are polymeric moisturizers of the familiy of water soluble and/or with water gelating polysaccarides such as hyaluronic acid, chitosan and/or fucose rich polysaccharides available, e.g., as Fucogel1000 (CAS-Nr. is 178463-23-5) from SOLABIA S. The moisturizing agent is optionally present in an amount varying from about 0.5 to about 8%, preferably from about 1 to about 5% of the total weight of the composition.

Suitable neutralizing agents which may be included in the composition of the present invention to neutralize components such as e.g. an emulsifier or a foam builder/stabilizer include but are not limited to alkali hydroxides such as a sodium and potassium hydroxide) organic bases such as diethanolamine (DEA), triethanolamine (TEA), aminomethyl propanol, trisodium ethylenediaminetetraacetic acid and mixtures thereof; basic amino acids such as arginine and lysine and any combination of any of the foregoing. The neutralizing agent may be present in an amount of about 0.01 to about 8% in the compositions of the present invention, preferably 1 to about 5%. The addition of electrolytes into the composition of the present invention may be necessary to change the behavior of a hydrophobic emulsifier. Thus the emulsions may preferably contain electrolytes of one or several salts including anions such as a chloride, a sulfate, a carbonate, a borate or an aluminate, without being limited thereto. Other suitable electrolytes may be on the bases of organic anions such as, but not limited to, lactate, acetate, benzoate, propionate, tartrate and citrate. As cations preferred are ammonium, alkyl ammonium, alkaline or alkaline earth metals such as Sodium or Magnesium. Especially preferred salts are potassium and sodium chloride, magnesium sulfate, zinc sulfate and mixtures thereof. Electrolytes are preferably present in an amount of about 0.01 to about 0.5% in the compositions of the present inventions.

According to another aspect of the invention there is provided a method of providing protection from UV-vis radiation comprising applying a composition of the invention to a desired external surface of the body.

According to another aspect of the invention there is provided use of a composition of the invention for providing an SPF higher than 15.

The use is preferably non-therapeutic.
The invention will now be explained in detail with help of the following non-limiting examples, which form preferred embodiments of the various aspects of the invention.

### Examples

### Examples 1-7: Compositions as per the invention comprising various polymers within and outside the invention.

Various compositions as shown in Table 1 were prepared.

**Table 1**

| Example | 1 | 2 | 3 | 4 | 5 | 6 | 7 |
|---|---|---|---|---|---|---|---|
| Ingredients | Wt% | Wt% | Wt% | Wt% | Wt% | Wt% | Wt% |
| Glycerine | 1 | 1 | 1 | 1 | 1 | 1 | 1 |
| KOH | 0.569 | 0.569 | 0.569 | 0.569 | 0.569 | 0.569 | 0.569 |
| Hystric acid | 5 | 5 | 17 | 5 | 17 | 5 | 5 |
| Cetyl alcohol | 0.53 | 0.53 | 0.53 | 0.53 | 0.53 | 0.53 | 0.53 |
| DC 200-350 | 0.5 | 0.5 | 0.5 | 0.5 | 0.5 | 0.5 | 0.5 |
| IPM | 1 | 1 | 1 | 1 | 1 | 1 | 1 |
| Parsol MCX | 2.25 | 2.25 | 2.25 | 2.25 | 2.25 | 2.25 | 2.25 |
| Parsol 1789 | 1.2 | 1.2 | 1.2 | 1.2 | 1.2 | 1.2 | 1.2 |
| Phenoxyeth anol | 0.2 | 0.2 | 0.2 | 0.2 | 0.2 | 0.2 | 0.2 |
| Titanium dioxide | 0.6 | 0.6 | 0.6 | 0.6 | 0.6 | 0.6 | 0.6 |
| Polymer | HEC | MC | PVA1 | Pluronic | PVA2 | SCMC | Cellulose gum |
| Polymer, wt% | 2.0 | 1.0 | 3.0 | 2.0 | 2.0 | 2.0 | 2.0 |
| Water | To 100 | To 100 | To 100 | To 100 | To 100 | To 100 | To 100 |

In the table above the acronyms stand for the following:
Hystric acid: Is a mixture of 45 % palmitic acid and 55 % stearic acid.
DC 200-350: Polydimethylsiloxanes/Dimethicones supplied by Dow corning
IPM: Iso propyl myrsitate.
Parsol MCX: A UV-B sunscreen with chemical name 2-ethylhexyl-4-methoxycinnamate supplied by DSM.
Parsol 1789: A UV-A sunscreen with chemical name 4-tert.-butyl-4'-methoxydibenzoylmethane supplied by DSM.
KOH: Potassium hydroxide.
HEC: Hydroxy ethyl cellulose.
SCMC: Sodium carboxymethyl cellulose.
MC: Methyl cellulose.
PVA1: is Polyvinyl alcohol with catalog number LH-05 from Gohsenol with 86.5-89% DH with a viscosity of 4.8 to 5.8 cP (for a 4% solution at 20 °C)
Pluronic has chemical family name Ethylene Oxide/Propylene Oxide Block Copolymer supplied by BASF.
PVA2: is poly vinyl alcohol with catalog number 36306-5 supplied by Sigma Aldrich with 125-185 K and 99% DH.

The compositions of Table -1 were measured for SPF using the following procedure and the data is summarized in Table - 2. The average SPF value reported is an average of three readings.

### In-vitro SPF Measurement

For the in vitro SPF measurements, 2mg/cm² of the formulation was applied in small dots at regular intervals on a 70 mm roughened PMMA plate-6µm roughness (Schonberg GmbH&Co.KG) at an application rate 2mg/cm² within 30 seconds. These dots were spread uniformly in an orderly manner using a parafilm as fingercot and the substrate with the formulation was dried for 15 minutes. SPF values were measured using an Optometrics SPF 290S system.

**Table 2**

| Example | Polymer (level) | HLB | Avg.SPF |
|---|---|---|---|
| 1 | HEC (2%) | 8.1 | 20.1 |
| 2 | MC (1%) | 7 - 10 | 26.2 |
| 3 | PVA1 (3%) | 15.2 - 16.2 | 31.6 |
| 4 | Pluronic (3%) | 16.5 | 34.3 |
| 5 | PVA2 (2%) | 19.6 | 12.6 |
| 6 | SCMC (2%) | 20 | 11.8 |
| 7 | Cellulose gum(2%) | 16.5 | 6.3 |

The data in Table 2 above indicates that polymers as per the invention having the required HLB values (Examples 1 to 4) provide for high SPF while those outside the invention provide low SPF values (Example 5 to 7).

### Examples 3,5 and 8-10: Effect of different types of polyvinyl alcohol

Various types of polyvinyl alcohols were used to study the effect of different types of such polymers on the composition of the invention as shown in Table 3. The compositions of Table -3 were measured for SPF using the same procedure as described earlier and the data is also summarized in Table 3.

**Table 3**

| Example | Polymer | HLB | Avg.SPF |
|---|---|---|---|
| 3 | PVA1 | 15.2-16.2 | 31.6 |
| 5 | PVA2 | 19.6 | 12.6 |
| 8 | PVA3 | 15.2 -16.1 | 18.75 |
| 9 | PVA4 | 15.3-16.2 | 18.23 |
| 10 | PVA5 | 19.6 | 15.14 |

In the table above the acronyms stand for the following:
PVA1: is Polyvinyl alcohol with catalog number LH-05 from Gohsenol with 86.5-89% DH with a viscosity of 4.8 to 5.8 cP (for a 4% solution at 20 °C)
PVA2: is poly vinyl alcohol with catalog number 36306-5 supplied by Sigma Aldrich with 125-185 K and 99% DH.
PVA3 is poly vinyl alcohol with catalog number 81381 supplied by Fluka with 31 K and 86.7 to 88.7% DH.
PVA4 is poly vinyl alcohol supplied by Sigma-Aldrich with 13-23 K and 86 to 89%% DH.
PVA5 is poly vinyl alcohol with catalog number 341584 supplied by Sigma-Aldrich with 89-98 K and 99% DH.

The data in Table 3 above indicates that even within the poly vinyl alcohol class, those within the HLB values claimed (Example 3, 8, 9) provide higher SPF values as compared to those outside the invention (Examples 5 and 10)

### Examples 11 to 14: Effect of amount of polymer

Polyvinyl alcohol PVA1 with catalog number LH-05 from Gohsenol with 86.5-89% DH with a viscosity of 4.8 to 5.8 cP (for a 4% solution at 20 °C) was used in composition similar to Example 3 except that the amount of PVA was varied as shown in Table 4 and the SPF of those compositions measured using the procedure as described earlier.

**Table 4**

| Example | Wt% Polymer | Avg.SPF |
|---|---|---|
| 11 | 0.0 | 11.6 |
| 12 | 0.5 | 15.4 |
| 13 | 2.0 | 22.4 |
| 14 | 4.0 | 32.8 |

The data in Table 4 above indicates that the polymer is essential to providing high SPF. Further polymer at 0.5 to 4% is preferred (Examples 12 to 14).

### Examples 15 - 19: Effect of amount of fatty acid

Various compositions similar to Example 9 were prepared as shown in Table 5 below except that the amount of fatty acid (hystric acid) was varied. The effect of the amount of fatty acid used on SPF is given below.

**Table 5**

| Example | Wt% fatty acid | Avg.SPF |
|---|---|---|
| 15 | 0.0 | 5.0 |
| 16 | 5.0 | 15.4 |
| 17 | 10.0 | 28.1 |
| 18 | 15.0 | 30.1 |
| 19 | 20.0 | 35.7 |

The data in the table 5 above indicates that a composition without any fatty acid (Example 15) does not give high SPF while those with fatty acid within the given range provides high SPF. It is preferred that the fatty acid is from 8 to 20% by weight of the composition.

## Claims

1. A sunscreen composition comprising at the most 12% organic sunscreens comprising
(i) 0.1 to 5% of a UVA sunscreen;
(ii) 0.1 to 10% of a UVB sunscreen;
(iii) 0.1 to 10% of a non-ionic linear polymer having a molecular weight higher than 8000 and HLB value from 7 to 18 selected from the group of poly vinyl alcohol, modified cellulose and poly(propylene oxide-co-ethylene oxide);
(iv) 4 to 25 % fatty acid; and
(v) 40 to 80% water,
**characterized in that**:
- the UVA sunscreen comprises a dibenzoylmethane, triazine, or triazone derivative; and
- the non-ionic linear polymer (iii) comprises poly vinyl alcohol.

2. A composition as claimed in claim 1 wherein said fatty acid is present in 8 to 20% by weight of the composition.

3. A composition as claimed in claim 1 or 2 wherein the total amount of organic sunscreen is at the most 8%.

4. A composition as claimed in any one of the preceding claims wherein the HLB of the non-ionic polymer is in the range of 15 to 18.

5. A composition as claimed in any one of the preceding claims wherein said non-ionic polymer is present in 0.5 to 4%.

6. A composition as claimed in any preceding claim 1 to 5 wherein said dibenzoylmethane derivative is 4-tert-butyl-4'-methoxydibenzoylmethane.

7. A composition as claimed in any one of the preceding claims wherein the UV-B sunscreen is selected from the class of benzophenones, anthranilates, salicylates, cinnamates, camphores, benzylidene malonate; triazone or derivatives thereof.

8. A composition as claimed in any one of the preceding claims wherein the surface tension of a 1% solution of the non-ionic polymer in water at 25 °C is higher than 40 dynes/cm.

9. A composition as claimed in any one of the preceding claims wherein the fatty acid is present in higher than 12% by weight of the composition.

10. A composition as claimed in any one of the preceding claims additionally comprising 0.1 to 10% soap.

11. a composition as claimed in any one of the preceding claims for use in providing an SPF value of at least 15.

## Patentansprüche

1. Sonnenschutzzusammensetzung, die höchstens 12% organische Sonnenschutzmittel umfasst, umfassend
(i) 0,1 bis 5% eines UVA-Sonnenschutzmittels,
(ii) 0,1 bis 10% eines UVB-Sonnenschutzmittels,
(iii) 0,1 bis 10% eines nicht-ionischen linearen Polymers, das ein Molekulargewicht höher als 8000 und einen HLB-Wert von 7 bis 18 aufweist, ausgewählt aus der Gruppe aus Polyvinylalkohol, modifizierter Cellulose und Poly(propylenoxid-co-ethylenoxid),
(iv) 4 bis 25% Fettsäure und
(v) 40 bis 80% Wasser,
**dadurch gekennzeichnet, dass**
- das UVA-Sonnenschutzmittel ein Dibenzoylmethan-, Triazin- oder Triazon-Derivat umfasst und
- das nicht-ionische lineare Polymer (iii) Polyvinylalkohol umfasst.

2. Zusammensetzung, wie im Anspruch 1 beansprucht, wobei die Fettsäure mit 8 bis 20 Gewichts-% der Zusammensetzung vorliegt.

3. Zusammensetzung, wie im Anspruch 1 oder 2 beansprucht, wobei die Gesamtmenge des organischen Sonnenschutzmittels höchstens 8% beträgt.

4. Zusammensetzung, wie in irgendeinem der vorhergehenden Ansprüche beansprucht, wobei der HLB des nicht-ionischen Polymers in dem Bereich von 15 bis 18 liegt.

5. Zusammensetzung, wie in irgendeinem der vorhergehenden Ansprüche beansprucht, wobei das nicht-ionische Polymer mit 0,5 bis 4% vorliegt.

6. Zusammensetzung, wie in irgendeinem der vorhergehenden Ansprüche 1 bis 5 beansprucht, wobei das Dibenzoylmethan-Derivat 4-tert-Butyl-4'-methoxydibenzoylmethan ist.

7. Zusammensetzung, wie in irgendeinem der vorhergehenden Ansprüche beansprucht, wobei das UVB-Sonnenschutzmittel aus der Klasse von Benzophenonen, Anthranilaten, Salicylaten, Cinnamaten, Kampfern, Benzylidenmalonat, Triazon oder Derivaten davon ausgewählt ist.

8. Zusammensetzung, wie in irgendeinem der vorhergehenden Ansprüche beansprucht, wobei die Oberflächenspannung einer 1%-igen Lösung des nicht-ionischen Polymers in Wasser bei 25°C höher als 40 Dyn/cm ist.

9. Zusammensetzung, wie in irgendeinem der vorhergehenden Ansprüche beansprucht, wobei die Fettsäure mit mehr als 12 Gewichts-% der Zusammensetzung vorliegt.

10. Zusammensetzung, wie in irgendeinem der vorhergehenden Ansprüche beansprucht, die zusätzlich 0,1 bis 10% Seife umfasst.

11. Zusammensetzung, wie in irgendeinem der vorhergehenden Ansprüche beansprucht, zur Verwendung bei der Bereitstellung eines SPF-Wertes von mindestens 15.

## Revendications

1. Composition d'écran solaire comprenant au plus 12 % d'écrans solaires organiques comprenant
(i) de 0,1 à 5 % d'un écran solaire UVA ;
(ii) de 0,1 à 10 % d'un écran solaire UVB ;
(iii) de 0,1 à 10 % d'un polymère linéaire non-ionique ayant une masse moléculaire supérieure à 8 000 et une valeur HLB de 7 à 18 choisi dans le groupe de poly(alcool vinylique), cellulose modifiée et poly(oxyde de propylène-co-oxyde d'éthylène) ;
(iv) de 4 à 25 % d'acide gras ; et
(v) de 40 à 80 % d'eau,
**caractérisée en ce que** :
- l'écran solaire UVA comprend un dibenzoylméthane, une triazine ou un dérivé de triazone ; et
- le polymère linéaire non-ionique (iii) comprend du poly(alcool vinylique).

2. Composition selon la revendication 1, dans laquelle ledit acide gras est présent dans de 8 à 20 % en masse de la composition.

3. Composition selon la revendication 1 ou 2, dans laquelle la quantité totale d'écran solaire organique est au plus de 8 %.

4. Composition selon l'une quelconque des revendications précédentes, dans laquelle le HLB du polymère non-ionique se trouve dans l'intervalle de 15 à 18.

5. Composition selon l'une quelconque des revendications précédentes, dans laquelle ledit polymère non-ionique est présent dans de 0,5 à 4 %.

6. Composition selon l'une quelconque des revendications 1 à 5 précédentes, dans laquelle ledit dérivé de dibenzoylméthane est le 4-tert-butyl-4'-méthoxydibenzoylméthane.

7. Composition selon l'une quelconque des revendications précédentes, dans laquelle l'écran solaire UV-B est choisi dans la classe des benzophénones, anthranilates, salicylates, cinnamates, camphres, malonate de benzylidène ; triazone ou dérivés de ceux-ci.

8. Composition selon l'une quelconque des revendications précédentes, dans laquelle la tension superficielle d'une solution à 1 % du polymère non-ionique dans l'eau à 25°C est supérieure à 40 dynes/cm.

9. Composition selon l'une quelconque des revendications précédentes, dans laquelle l'acide gras est présent dans plus de 12 % en masse de la composition.

10. Composition selon l'une quelconque des revendications précédentes, comprenant de plus de 0,1 à 10 % de savon.

11. Composition selon l'une quelconque des revendications précédentes pour une utilisation dans la fourniture d'une valeur SPF d'au moins 15.
